# EUROPEAN PATENT APPLICATION

(11) **EP 1 741 420 A1**
(43) Date of publication of application: **10.01.2007**
(21) Application number: 05014750.3
(22) Date of filing: 07.07.2005
(51) Int. Cl.: A61K 6/10, A61K 6/093

(54) **Quick set silicone based dental impression materials**

(71) Applicant: Coltène AG, 9450 Altstätten (CH)
(72) Inventor: Kollefrath, Ralf, Dr., 9464 Rüthi (CH); Benz, Silvan, 9464 Rüthi (CH)
(74) Representative: Welch, Andreas

(57) **Abstract**

The invention relates to a multi-component addition-cured silicon impression compound, comprising at least one organopolysiloxane comprising at least two unsaturated groups in the molecule; at least one organohydrogenpolysiloxane comprising at least three SiH groups in the molecule; at least one platinum catalyst and at least one filler.

## Description

### Background

In prosthodontics, impression materials of various types are used to form a mold of teeth or other oral structures. Dental impression materials are commonly composed of silicone materials that are stored until use in the dental office in a semi-finished manner. When required for use the two [or more] components of the impression material are combined, setting off a chemical reaction which in a short period of time changes the flowable character of the material to a hardened material. When the flowable material is applied to a structure such as a tooth it will harden to form an exact impression of that structure. The formed impression material can then be used in any number of conventional purposes.

Platinum catalyzed two component addition cure silicones are high tear strength, flexible, mold compounds. These silicones are, for example, modified polydimethylpolysiloxanes, as disclosed, for example in USP 4,035,453, whose disclosure is incorporated herein and made a part hereof. One component contains a pre-polymer in which some of the methyl groups in the polydimethylpolysiloxanes are replaced by hydrogen. The other component contains a pre-polymer in which some of the methyl groups in the polydimethylpolysiloxanes are replaced by vinyl groups. A catalyst, typically a platinum catalyst, is present in the second component. When the two components are brought into contact crosslinking [polymerization] occurs at a relatively rapid rate. During an initial working period, the components are readily mixed and the material remains relatively fluid. As polymerization occurs the viscosity of the mixture increases [setting period] until it is complete and the silicone rubber is no longer fluid in typical use.

In the dental office it is desirable to use materials that have a reasonably long working time so as to permit the proper mixing and application of the material to the dental structure whose impression is desired but which have a relatively short setting time so that once the material is applied it quickly sets, saving the dentist time and the patient discomfort.

One technique for increasing the working time has been to add a retarder to the mixture. Use of retarders with light-bodied addition silicones do increase the working time in many instances but result in degraded impressions and poorly fitted crowns fabricated from the impression. The compression set of retarded mixes reportedly increased four fold in some instances when removed at the recommended removal time and only an increase of removal time of approximately a minute resulted in a satisfactory compression set.

A formulation would be desirable that utilized a retarder in varying amounts but did not cause poor compression set or require extended setting times.

Thus, there is a two fold need existing in present dental impression materials - a need to provide an adequate, or even extended, working period and a very quick setting period.

### Summary

One aspect of the invention is a two component silicone based impression material containing a multi-variant catalyst/crosslinker/retarder system utilizing a new retarder that meets the requirements of a dental impression material with flexible working and setting times. In particular, the new formulation significantly increases the rapidity of setting and permits a significant shortening of the time required to obtain an impression.

In one embodiment, a two component silicone based impression material is provided that has a working time that can be modified to suit the requirements of the dentist while retaining a short setting time.

In two other preferred embodiments, the new two component silicone based impression material can be formulated to have a working time equal to that of conventional silicone impression materials and a shorter overall time from mixing to setting or the two component silicone based impression material can be formulated to provide an equivalent overall time as with conventional materials, in conjunction with a longer working time.

Another aspect of the invention provides improved compositions based on the discovery that the working time, setting time, and therefore the overall duration of the operation from start to recovery of a formed impression of an oral structure can be modified in a controlled manner by varying four components of the impression material formulation: 1] the SiH/vinyl ratio, 2] the number of SiH groups in the crosslinker 3] the amount of catalyst and 4] the amount of an alkynol/alkyne retarder.

### Figures

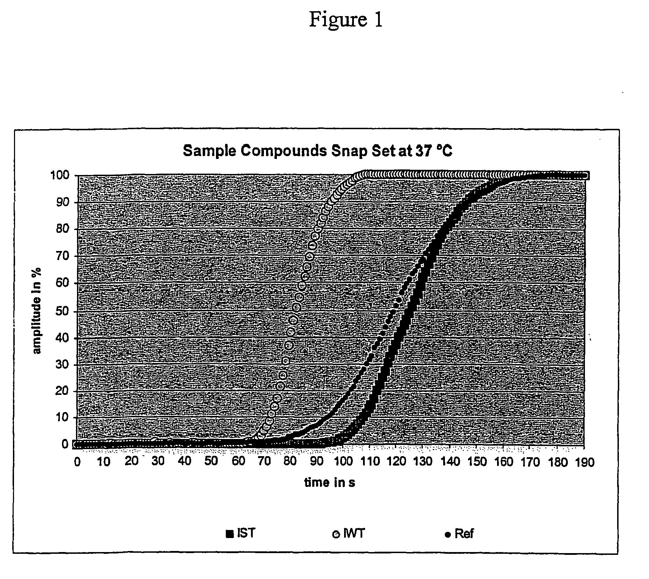

Figure 1 is a graph comparing the working and setting times of a prior art formulation (Ref) with two formulations of the disclosed formulation according to the invention (IST and IWT).

### Detailed Description

The multi-variant impression matérial utilizing an alkynol and/or alkyne retarder comprises a hydrogen modified polydimethylsiloxane, a vinyl modified polydimethylsiloxane, a catalyst, and a retarder; it may further comprise a crosslinker, filler and, optionally, other additives.

The formulation preferably is a multi-component, preferably two-component addition-crosslinking silicone impression material. One component, typically referred to as "base", contains a silicone which has a number of the methyl groups substituted by hydrogen atoms. This first component also comprises at least one organopolysiloxane having at least two unsaturated groups in the molecule, at least one filler and an alkynol/alkyne retarder.

Prior to mixing, in a two component system, a first component preferably comprises, in weight percent of the first component:
from about 15 to about 60% by weight of at least one organopolysiloxane having at least two unsaturated groups in the molecule,
from about 1 to about 15% by weight of at least one organohydrogenpolysiloxane having at least three 3 SiH groups in the molecule, preferably three [3] to fifteen [15], more preferably four [4] to eleven [11], most preferably five [5] to eight [8].
from about 3 to about 80% by weight of at least one filler,
from about 0.005 to about 0.05 % by weight of an alkynol or alkyne,
with this component exhibiting a viscosity in the range of from 0.5 to 500 Pa·s.

The second component, typically referred to as "catalyst" comprises a silicone material which -has several of its methyl groups replaced by vinyl groups. Also present in the second component are at least one platinum catalyst and one or more fillers.

Prior to mixing, in a two component system, a second component preferably comprises, in weight percent of the second component
from about 20 to about 60% by weight of at least one organopolysiloxane having at least two unsaturated groups in the molecule,
from about 0.005 to about 0.01 % by weight of at least one hydrosilylation catalyst, preferably at least one platinum catalyst, most preferably a Pt(0) complex (calculated as elemental metal),
from about 3 to about 80% by weight of at least one filler

with this component exhibiting a viscosity in the range of from 0.5 to 500 Pa·s.

In one embodiment, the two components are employed in a volume ratio which is in the range from about 1:1 to about 1:100, more preferably in the range from about 1:1 to about 1:10 and particularly preferably in the range from about 1:1 to about 1:2.

After mixing, the composition comprises, in weight percent of the total composition:

| Component | Workable Range | Preferred | Most Preferred |
|---|---|---|---|
| Hydrosilylation catalyst | 1 - 200 ppm | 20-150 ppm | 50-100 ppm |
| Retarder | 1 - 500 ppm | 20 - 350 ppm | 50 - 200 ppm |
| Filler | 3-80% | 25-75% | 35-70% |
| Polydialkylsiloxanes (total) | 97 - 20 % | 75-25% | 65-30% |
| including divinylpolydialkylsiloxanes and crosslinkers in a ratio SiH / Vinyl of | 1.00-2.50 | 1.30-2.20 | 1.50 - 1.80 |
| Additives (pigments, surfactants, modifiers | variable | variable | variable |

One or more linear diorganovinylsiloxy-terminated polydiorganosiloxanes, preferably one or more linear dimethylvinylsiloxy-terminated polydimethylsiloxanes having differing viscosities are preferred in certain embodiments of the invention.

### Silicones

The organopolysiloxane having hydrogen moieties in the molecule can consist of one or more organopolysiloxanes which differ from each other. It is likewise possible for the organopolysiloxane having vinyl groups to contain one or more organopolysiloxanes which differ from each other. Preferred unsubstituted diorganopolysiloxanes of this structure are depicted by the following formula

CH₂ =CH(-SiR₂-O)ₙ-SiR₂-CH=CH₂

where R represents an unsubstituted or substituted monovalent hydrocarbon group which is preferably free from aliphatic multiple bonds, and n represents an integer and exhibits a Brookfield viscosity in the range from 0.05 to 1000 Pa·s, characterized in that, in the mixed state, the silicone impression material exhibits a consistency according to one of the four types defined in ISO 4823:2000.

In a preferred embodiment, at least 50% of the radicals R are methyl groups. R may also be ethyl, vinyl, phenyl or 3,3,3-trifluoropropyl groups. The value ofn is preferably selected such that the polymer has a viscosity at 25°C which is in the range of from 25 to 500,000 mPa·s. Molecules of this nature are well known in the art and are described, for example, in USP 4,035,453, the entire disclosure of which is incorporated by reference into the present application.

The silicones are prepared using customary methods which are described, for example, in W. Noll, "Chemie und Technologie der Silikone" (Chemistry and Technology of the Silicones)", Verlag Chemie Weinheim, 2nd Edition 1964, pages 162-206.

The composition of an impression material can vary in a very broad range depending on the physical properties wanted such as low viscous light body vs. high viscous putty. For this reason the ranges provided herein are given for the most typical range of properties desired by dental practitioners and are not intended to be limit the invention to solely those ranges.

A crosslinker is an organopolysiloxane having at least three Si-bonded hydrogen atoms per molecule. Suitably, this organopolysiloxane contains from 0.01 to 1.7 % by weight of Si-bonded hydrogen atoms, and the silicon valences not satisfied by hydrogen atoms, or siloxane oxygen atoms are satisfied by unsubstituted or substituted monovalent hydrocarbon radicals free of aliphatic multiple bonds, at least 50 percent of the hydrocarbon radicals bonded to silicon atoms carrying hydrogen atoms being methyl radicals.
The SiH-concentration per module is at least three [3], preferably three [3] to fifteen [15], more preferably four [4] to eleven [11], most preferably five [5] to eight [8]. **Retarders**

The most commonly utilized prior art retarders are 1,3,5,7-tetravinyl-tetramethylcyclotetrasiloxane and 1,3-divinyltetramethyldisiloxane (DVTMDS). This compound is added to the formulation as such, or it may be combined with a platinum to form Karstedt's catalyst (Pt/DVTMDS) or it may be added in both forms. Although it is possible to vary the working time of the formulation through the use of prior art retarders and other components, the setting time is not greatly variable with prior art retarders.

The preferred new retarder compounds are various alkynes or alkynols that allow a substantial shortening of the setting time. They react with the SiH moieties in the formulation and are fixed into the polymer. The preferred retarders are alkynols having the formula R¹-C(OH)-C≡C-R² where R¹ may be 1 to 20 carbons, branched, cyclic, linear, or combinations thereof, preferably where R¹= C₁₋₁₅, most preferably where R¹ = C₂₋₁₀ and R² may be hydrogen or C₁₋₂. This is approximately the widest workable range because as the chain increases in length and molecular weight, the materials become solid and are more difficult to dissolve or disperse into the silicone. A currently preferred alkynol for use in the present invention is ethyl octynol, CH₃(CH₂)₃CH(C₂H₅)CH(OH)C≡CH.

The alternatives are alkynes of the formula R¹-C≡C-R² with the same carbon chain limits.

Retarder concentrations range from about 1 to about 500 ppm, preferably form about 20 to about 350 ppm and most preferably from about 50 to about 200 ppm in the impression material.

Surprisingly, the compositions according to the invention allow for a great variety of reaction profiles (and resulting working times and setting times, respectively) to be adjusted, without any further heating step being necessary in order to allow the composition to cure.

### Catalyst

In principle any catalyst suitable for hydrosilylation reactions at room temperature known to the experienced formulator will be suitable. Most common are catalysts of the platinum metals group, preferably platinum catalysts such as hexachloroplatinic acid and Karstedt-catalyst.

For at least the two common catalysts (hexachloroplatinic acid and Karstedt-catalyst) it is common to utilize the catalyst in an amount in the range from 1 to 200 ppm, preferably from 20 to 150 ppm, and particularly preferably in a range from 50 to 100 ppm.

In one technique for varying the proportions of the components most active in determining working and setting time, the catalyst is held to a level of approximating 100 ppm of the final composition. Utilizing this amount of catalyst as a constant, setting behavior can then be modified within desired limits by varying the final formulation, the appropriate amounts of the retarders according to the invention, the ratio of vinyl- and SiH-groups and/or the total amount of vinyl groups in the formulation.

A change between the hexachloroplatinic acid and Karstedt-catalyst results in a linear relationship (amount of platinum), however due to the handling and solubility advantages Karstedt-catalyst is widely preferred in the dental industry.

### Fillers

Fumed pyrogenic silica is an exceptionally pure form of silicon dioxide manufactured by heating silicon tetrachloride in an oxy-hydrogen flame. Particle sizes typically range from 0.0007 to 0.05 µm. When used in a composition with polydiorganosiloxanes it interacts strongly with the siloxane chain and can increase the tensile strength of the resulting polymer by a factor of as much as ten.

Fillers which are suitable for use in the invention are either non-reinforcing fillers having a BET surface of up to 50 m²/g, such as quartz, cristobalite, calcium silicate, zirconium silicate, montmorillonites, bentonites, zeolites including molecular sieves, metal oxide powders, barium sulfate, calcium carbonate, gypsum and glass and plastic powders. Quartz, cristobalite and sodium aluminum silicates, especially when surface-treated, are preferred.

While the fillers may have a surface are up to about 50 m²/g it is preferred that the fillers exhibit a BET surface area of up to 15 m²/g.

Other fillers, such as reinforcing fillers having a BET surface area of greater than 50 m²/g, preferably up to about 15 m²/g such as pyrogenic or precipitated silicic acid or silicon aluminum mixed oxides are also suitable for use.

Both reinforcing and nonreinforcing fillers may be utilized, with, for example, at least one reinforcing filler being present in one of the components and at least one nonreinforcing filler being present in the other component. It is likewise also possible for reinforcing and nonreinforcing filler to be present together in one single component or to be present in both components, with it being possible for reinforcing fillers which are different from each other, or different nonreinforcing fillers, to be present in the different components.

The fillers can be surface-treated and, in this connection, preferably hydrophobized, for example by being treated with organosilanes or organosiloxanes or by hydroxyl groups being etherified to give alkoxy groups. The fillers are present in the impression material in a proportion in the range from 0 to 80% by weight, preferably in a range from 25 to 75% by weight and particularly preferably in a range from 30 to 65% by weight of the total composition.

### Surfactants

Surfactants are a useful component in the formulation to reduce the surface tension of the components and to facilitate mixing. The formulation must be able to displace moisture on the tooth and should make intimate contact with the structure whose image is being obtained. Increased wettability through the use of surfactants is highly desirable. A useful surfactant is TERGITOL NP-4^{™} manufactured by DOW.

The working time and setting time is measured by a technique showing the increase of viscosity by developing elastic behavior. Amplitude is the amplitude of the oscillating rheometer used for the measurements. All the amplitudes are normalized to a maximum of 100%.

### Examples

The following chart records the results obtained when the prior art formulation is compared to the newly disclosed formulation (WT = working time; ST,= setting time).

| | **Example 1 Prior Art** | | **Example 2 Identical ST (Setting Time)** | | **Example 3 Identical WT (Working Time)** | |
|---|---|---|---|---|---|---|
| **Component/Time** | WT = 149 s | ST = 179 s | WT=210 s | ST = 175 s | WT= 140 s | ST =117 s |
| | Base in g | Catalyst in g | Base in g | Catalyst in g | Base in g | Catalyst in g |
| Divinylpolydimethylsiloxane (1 Pas, 0.14 mmol/g Vinyl) [Silopren U1, GE Bayer] | 15.000 | 17.950 | 13.496 | 17.955 | 13.499 | 17.955 |
| Divinylpolydimethylsiloxane (0.2 Pas, 0.3 mmol/g Vinyl) [Silopren U0.2, GE Bayer] | 2.500 | 2.500 | | 2.501 | | 2.501 |
| Crosslinker (40 mPas, 4.2 mmol/g SiH, 13 SiH) [Silopren U Vernetzer 430, GE Bayer] | 2.500 | | | | | |
| Crosslinker (60 mPas, 1.8 mmol/g SiH, 7 SiH) [experimental] | | | 6.498 | | 6.499 | |
| Quartz powder, silanized [Silbond 8000 RST, Quarzwerke] | 30.000 | 27.265 | 29.991 | 27.273 | 29.997 | 27.273 |
| Pt-catalyst (0.15% Pt) [Karstedt-catalyst diluted in Silopren U1/quartz filler, made in house] | | 2.270 | | 2.271 | | 2.271 |
| DVTMDS [Divinyltetramethyldisiloxane, Aldrich] | | 0.015 | | | | |
| Alkynol [Ethyloctynol, BASF] | | | 0.015 | | 0.005 | |
| Total | 50.000 | 50.000 | 50.000 | 50.000 | 50.000 | 50.000 |
| | | | | | | |
| | WT measured at 23 °C, ST at 37 °C; WT can be longer than ST | | | | | |

In example 1 the crosslinker is a polydimethylsiloxane in which 13 of the methyl groups have been substituted by hydrogen. The Pt-catalyst paste is a catalyst paste obtained from Karstedt-catalyst diluted with Silopren U1 a vinyl modified dimethypolysiloxane [GE Bayer Silicones, Leverkussen, Germany], / quartz filler. The DVTMDS is obtained from Aldrich.

In example 2 the crosslinker is a polydimethylsiloxane in which 7 of the methyl groups have been substituted by hydrogen. The retarder is ethyloctynol obtained from BASF. The setting time is identical to example 1, the working time is prolonged by almost a minute or 40%. This demonstrates flexibility of the system and the additional working time permitted while obtaining the same setting time.

In example 3 the amount of retarder compared to example 2 is varied, so the working time is identical to example 1, the setting time is reduced by almost a minute or 33%. This system allows faster impression taking however no compromising with working time.

As can be seen by these simple examples substitution of the alkynol for the conventional retarder causes a reduction of the setting time, as illustrated in Figure 1 where the steepness of the curve during setting clearly illustrates the time saving effect ("snap set") of substituting the alkynol /alkyne retarder for the conventional DVTMDS retarder.

Variable concentrations of retarder and crosslinker do not relate in simple accordance to a mathematical law which can predict the working and setting time behavior of a formulation. It will be understood that various modifications of the variables of vinyl/SiH ratio, the number of SiH groups in the crosslinker, catalyst concentration and alkynol/alkyne retarder will provide equivalent results and other modifications will provide results in various portions of the disclosed ranges as desired by the practitioner.

The degree of flexibility inherent in the disclosed formulations was not heretofore available to the field and provides a significant benefit to dental practitioners.

## Claims

1. A silicone material, preferably a dental impression composition, obtainable from a mixture comprising:
(i) at least one organopolysiloxane having at least two unsaturated groups in the molecule;
(ii) at least one organohydrogenpolysiloxane having at least-threeSiH groups in the molecule, Preferably three to five, more preferably four to eleven, most preferably five to eight;
(iii) a hydrosilylation catalyst; and
(iv) a retarder chosen from the group consisting of alkynols, preferably of the general formula R¹-C(OH)-C=C-R²; and alkynes, preferably of the general formula R¹-C=C-R²; wherein R¹ may be a C₁₋₂₀alkyl group and R² may be hydrogen or a C₁₋₂ alkyl group.

2. A silicone material according to claim 1 **characterized in that** the at least one organopolysiloxane (i) is or comprises at least one vinyl-modified polydimethylsiloxane.

3. A silicone material according to one of claims 1 or 2, **characterized in that** the at least one organohydrogenpolysiloxane (ii) is or comprises at least one hydrogen-modified polydimethylsiloxane.

4. A silicone material according to one of claims 1 to 3 **characterized in that** it is a multi-component, preferably a two-component material.

5. A silicone material according to claim 4, **characterized in that** a first component of the two-component material comprises:
from about 15 to about 60% by weight of the at least one organopolysiloxane having at least two unsaturated groups in the molecule;
from about 1 to about 15% by weight of the at least one organohydrogenpolysiloxane having at leasfthreeSiH groups in the molecule, preferably three to fifteen, more preferably four to eleven, most preferably five to eight;
from about 3 to about 80% by weight of at least one filler;
from about 0.005 to about 0.05% by weight of the retarder chosen from the group consisting of alkynols, preferably of the general formula R¹-C(OH)-C≡C-R²; and alkynes, preferably of the general formula R¹- C≡C-R²; wherein R¹ may be a C₁₋₂ (alkyl group and R² may be hydrogen or a C₁₋₂ alkyl group.

6. A silicone material according to claim 5 **characterized in that** said first component of the two-component material exhibits a viscosity in the range of from about 0.5 to about 500 Pa*s,

7. A silicone material according to one of claims 4 to 6, **characterized in that** a second component of the two-component material comprises:
from about 20 to about 60% by weight of the at least one organopolysiloxane having at least two unsaturated groups in the molecule;
from about 3 to about 80% by weight of at least one filler;
from about 0.005 to about 0.01% by weight of the hydrosilylation catalyst.

8. A silicone material according to claim 7, **characterized in that** said second component of the two-component material exhibits a viscosity in the range of from about 0.5 to about 500 Pa*s.

9. A silicone material according to one of claims 1 to 8, **characterized in that** said hydrosilylation catalyst is chosen from the group consisting of catalysts comprising elements of the platinum metals group, preferably platinum, most preferably Pt(0) complexes.

10. A silicone material according to claim 9, **characterized in that** the hydrosilylation catalyst is a Pt(0) complex with vinylsiloxan ligands.

11. Use of an alkynol, preferably of the general formula R¹-C(OH)-C=C-R²; and/or an alkyne, preferably of the general formula R¹-C=C-R²; wherein R¹ may be a C₁₋₂₀alkyl group and R² may be hydrogen or a C₁₋₂ alkyl group, as a retarder in a dental impression composition.

12. A method of reducing the setting time of a dental silicone material while maintaining the working time of said dental silicone material; and/or extending the working time of a dental silicone material while maintaining the setting time of said dental silicone material, **characterized in that** an alkynol, preferably of the general formula R¹-C(OH)-C≡C-R²; and/or an alkyne, preferably of the general formula R¹-C=C-R², wherein R¹ may be a C₁₋₂₀ alkyl group and R² may be hydrogen or a C₁₋₂ alkyl group, is added to the dental silicone material.
